# FASCICULE DE BREVET EUROPEEN

(11) **EP 1 294 689 B1**
(45) Date de publication et mention de la délivrance du brevet: **26.04.2006**
(21) Numéro de dépôt: 01954059.0
(22) Date de dépôt: 06.07.2001
(51) Int. Cl.: C07D 209/42, A61K 31/404, A61P 9/00

(54) **PROCEDE DE PREPARATION D'UNE NOUVELLE FORME CRISTALLINE BETA DU SEL DE TERT-BUTYLAMINE DU PERINDOPRIL**
VERFAHREN ZU DER HERSTELLUNG EINER NEUEN KRISTALLINEN FORM BETA DES TERT-BUTYLAMIN SALZES VON PERINDOPRIL
PREPARATION METHOD OF A NOVEL BETA CRYSTALLINE FORM OF PERINDOPRIL TERT-BUTYLAMINE SALT

(30) Priorité: 06.07.2000 FR 0008792
(43) Date de publication de la demande: 26.03.2003
(73) Titulaire: Les Laboratoires Servier, 92415 Courbevoie Cedex (FR)
(72) Inventeur: PFEIFFER, Bruno, F-95320 Saint Leu la Forêt (FR); GINOT, Yves-Michel, F-45000 Orleans (FR); COQUEREL, Gérard, F-76520 Boos (FR); BEILLES, Stéphane, F-21100 Dijon (FR)
(86) Numéro de dépôt international: PCT/FR2001/002168
(87) Numéro de publication internationale: WO 2001/087836

(56) Documents cités:
- EP-A- 0 308 341
- FR-A- 2 771 010

## Description

La présente invention concerne un procédé de preparation de la forme cristalline β du sel de tert-butylamine du perindopril de formule (I):

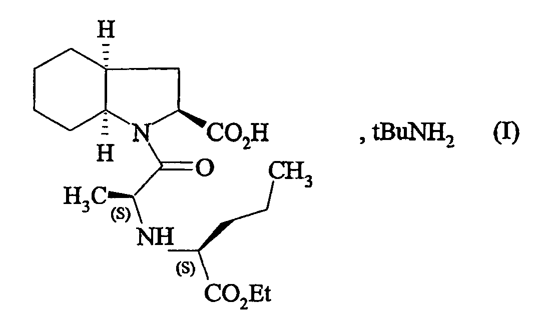

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine 1 (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte tenu de l'intérêt pharmaceutique de ce composé, il était primordial de l'obtenir avec une excellente pureté. Il était également important de pouvoir le synthétiser selon un procédé facilement transposable à l'échelle industrielle, et notamment sous une forme permettant une filtration et un séchage rapides. Enfin, cette forme devait être parfaitement reproductible, facilement formulée et suffisamment stable pour autoriser son stockage prolongé sans conditions particulières de température, de lumière, d'humidité ou de taux d'oxygène.

Le brevet EP 0 308 341 décrit un procédé de synthèse industrielle du perindopril. Cependant, ce document ne précise pas les conditions d'obtention du perindopril sous une forme présentant ces caractéristiques de manière reproductible.

La demanderesse a présentement trouvé qu'un sel particulier du perindopril, le sel de tert-butylamine, pouvait être obtenu sous une forme cristalline bien définie, parfaitement reproductible et présentant notamment des caractéristiques intéressantes de formulation.

Plus spécifiquement, la présente invention concerne un procédé de préparation de la forme cristalline β du composé de formule (I), caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, d'intensité et d'intensité relative (exprimée en pourcentage par rapport à la raie la plus intense) :

| **Angle 2 thêta (°)** | **Distance inter-réticulaire d (Å)** | **Intensité Intensité** | **Intensité relative (%)** |
|---|---|---|---|
| 5,169 | 17,08 | 523 | 16,5 |
| 8,379 | 10,54 | 1001 | 31,5 |
| 9,350 | 9,45 | 3175 | 100 |
| 14,746 | 6,00 | 236 | 7,4 |
| 15,411 | 5,74 | 753 | 23,7 |
| 15,931 | 5,56 | 279 | 8,8 |
| 16,711 | 5,30 | 113 | 3,6 |
| 18,161 | 4,88 | 122 | 3,8 |
| 20,564 | 4,32 | 1198 | 37,7 |
| 21,285 | 4,17 | 330 | 10,4 |
| 21,781 | 4,08 | 317 | 10 |
| 22,632 | 3,93 | 190 | 6 |
| 23,308 | 3,81 | 133 | 4,2 |
| 23,797 | 3,74 | 427 | 13,4 |
| 24,276 | 3,66 | 118 | 3,7 |

| | | | |
|---|---|---|---|
| 25,190 | 3,53 | 92 | 2,9 |
| 25,924 | 3,43 | 251 | 7,9 |
| 26,646 | 3,34 | 250 | 7,9 |
| 27,620 | 3,23 | 96 | 3 |
| 28,306 | 3,15 | 133 | 4,2 |

L'invention concerne un procédé de préparation de la forme cristalline β du composé de formule (I), caractérisé en ce que on porte à reflux une solution du sel de tert-butylamine du perindopril dans le dichlorométhane, puis on refroidit rapidement la solution à 0°C et collecte le solide obtenu par filtration.
- Dans le procédé de cristallisation selon l'invention, on peut utiliser le composé de formule (I) obtenu par n'importe quel procédé. Avantageusement, on utilise le composé de formule (I) obtenu par le procédé de préparation décrit dans le brevet EP 0 308 341.
- la concentration du composé de formule (I) dans le dichlorométhane est préférentiellement comprise entre 100 et 200 g/l.

Les exemples suivants illustrent l'invention mais ne la limitent en aucune façon.

Le spectre de diffraction X sur poudre a été mesuré avec les conditions expérimentales suivantes :
- Diffractomètre Siemens D5005, détecteur à scintillations,
- Anticathode de cuivre (λ=1,5405 Å), voltage 40 KV, intensité 40mA,
- Montage *θ*-*θ*,
- Domaine de mesures : 5° à 30°,
- Incrémentation entre chaque mesure : 0,02°,
- Temps de mesure par pas : 2s,
- Fentes variables : v6,
- Filtre K*β* (Ni),
- Pas de référence interne,
- Procédure de zéro avec les fentes Siemens,
- Données expérimentales traitées avec le logiciel EVA (version 5.0).

### EXEMPLE 1 : Forme cristalline β du sel de tert-butylamine du perindopril

135 g du sel de tert-butylamine du perindopril obtenu selon le procédé décrit dans le brevet EP 0 308 341 sont dissous dans 1100 ml de dichlorométhane portés au reflux. La solution est ensuite refroidie à 0°C et le solide obtenu est collecté par filtration.

### Diagramme de diffraction X sur poudre :

Le profil de diffraction des rayons X de la poudre (angles de diffraction) de la forme β du sel de tert-butylamine du perindopril est donné par les raies significatives rassemblées dans le tableau suivant, avec l'intensité et l'intensité relative (exprimée en pourcentage par rapport à la raie la plus intense) :

| **Angle 2 thêta (°)** | **Distance inter-réticulaire d (Å)** | **Intensité Intensité** | **Intensité relative (%)** |
|---|---|---|---|
| 5,169 | 17,08 | 523 | 16,5 |
| 8,379 | 10,54 | 1001 | 31,5 |
| 9,350 | 9,45 | 3175 | 100 |
| 14,746 | 6,00 | 236 | 7,4 |
| 15,411 | 5,74 | 753 | 23,7 |
| 15,931 | 5,56 | 279 | 8,8 |
| 16,711 | 5,30 | 113 | 3,6 |
| 18,161 | 4,88 | 122 | 3,8 |
| 20,564 | 4,32 | 1198 | 37,7 |
| 21,285 | 4,17 | 330 | 10,4 |
| 21,781 | 4,08 | 317 | 10 |
| 22,632 | 3,93 | 190 | 6 |
| 23,308 | 3,81 | 133 | 4,2 |
| 23,797 | 3,74 | 427 | 13,4 |
| 24,276 | 3,66 | 118 | 3,7 |
| 25,190 | 3,53 | 92 | 2,9 |
| 25,924 | 3,43 | 251 | 7,9 |
| 26,646 | 3,34 | 250 | 7,9 |
| 27,620 | 3,23 | 96 | 3 |
| 28,306 | 3,15 | 133 | 4,2 |

## Revendications

1. Procédé de préparation de la forme cristalline β du sel de tert-butylamine du perindopril de formule (I) : 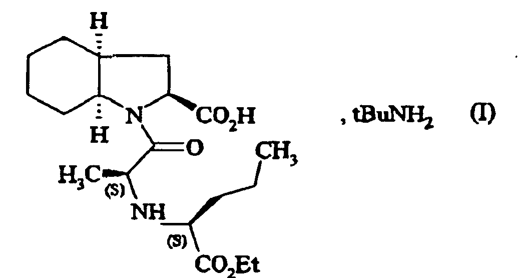 **caractérisé en ce que** l'on porte à reflux une solution du sel de tert-butylamine du perindopril dans le dichlorométhane, puis on refroidit la solution à 0°C et collecte le solide obtenu par filtration.

2. Procédé selon la revendication 1, **caractérisé en ce que** le composé de formule (I) qui est utilisé est obtenu par le procédé suivant:
- d'une part, réduction de l'ester éthylique de l'acide indole-2-carboxylique par le couple étain / acide chlorhydrique, suivie par une réaction de saponification, pour conduire à l'acide indoline-2-carboxylique racémique, dont l'isomère (S) est isolé par addition du racémate à une solution de (+)-α-méthylbenzylamine dans l'éthanol, pour obtenir un précipité du sel de l'acide (S)-indoline-2-carboxylique avec l'α-méthylbenzylamine, qui, après filtration, est dissous dans l'eau et acidifié, pour conduire à l'acide (S)-indoline-2-carboxylique, lequel, après filtration et lavage, est soumis à hydrogénation en présence de rhodium sur charbon, sous une pression d'hydrogène de 30 bars, en chauffant à une température de 60°C, pour conduire, après cristallisation, à l'acide (2S, 3aS, 7aS)-perhydroindole-2-carboxylique optiquement pur, qui est mis en réaction avec l'alcool benzylique en présence d'acide para-toluènesulfonique, pour conduire à l'ester benzylique correspondant,
- d'autre part, la (S)-L-norvaline est estérifiée par l'éthanol, pour conduire au (S)-norvalinate d'éthyle, qui est condensé avec l'acide pyruvique sous une pression d'hydrogène de 30 bars, en présence de charbon palladié, pour conduire, après cristallisation, refroidissement et filtration, à la N-[(S)-1-carbethoxybutyl]-(S)-alanine optiquement pure,
puis l'ester benzylique de l'acide (2S, 3aS, 7aS)-perhydroindole-2-carboxylique et la N-[(S)-1-carbéthoxybutyl]-(S)-alanine ainsi obtenus sont condensés en milieu alcalin, en présence de dicyclohexylcaibodiimide et de 1-hydroxybenzotriazole, pour conduire à l'amide correspondant, qui est soumis à déprotection du groupement carboxylique de l'hétérocycle, salification à l'aide de tert-butylamine, et cristallisation.

3. Procédé selon la revendication 1, **caractérisé en ce que** la concentration du composé de formule (I) dans le dichlorométhane est comprise entre 100 et 200 g/l.

## Patentansprüche

1. Verfahren zur Herstellung der β-Kristallform des tert.-Butylaminsalzes von Perindopril der Formel (I): 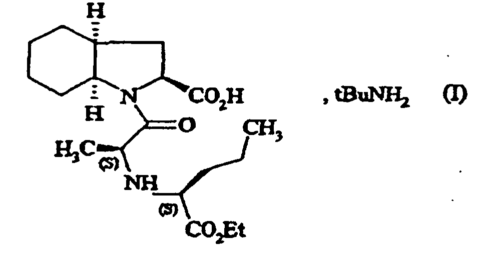 **dadurch gekennzeichnet, daß** man eine Lösung des tert.-Butylaminsalzes von Perindopril in Dichlormethan zum Sieden am Rückfluß erhitzt, dann die Lösung auf 0 °C abkühlt und den erhaltenen Feststoff durch Filtrieren abtrennt.

2. Verfahren nach Anspruch 1, **dadurch gekennzeichnet, daß** die verwendete Verbindung der Formel (I) mit Hilfe des folgenden Verfahrens hergestellt wird:
- einerseits Reduktion des Ethylesters der Indol-2-carbonsäure mit der Kombination Zinn/Chlorwasserstoffsäure, gefolgt von einer Verseifungsreaktion zur Bildung der racemischen Indolin-2-carbonsäure, deren (S)-Isomeres man isoliert durch Zugabe des Racemats zu einer Lösung von (+)-α-Methylbenzylamin in Ethanol zur Bildung eines Niederschlags aus dem Salz (S)-Indolin-2-carbonsäure mit α-Methylbenzylamin, welches man nach der Filtration in Wasser löst und ansäuert zur Bildung der (S)-Indolin-2-carbonsäure, welche man nach dem Filtrieren und Waschen bei einem Wasserstoffdruck von 30 bar und Erhitzen auf eine Temperatur von 60 °C in Gegenwart von Rhodium-auf-Kohlenstoff hydriert, so daß man nach der Kristallisation optisch reine (2S, 3aS, 7aS)-Perhydroindol-2-carbonsäure erhält, welche man in Gegenwart von p-Toluolsulfonsäure mit Benzylalkohol umsetzt zur Bildung des entsprechenden Benzylesters,
- andererseits (S)-L-Norvalin mit Ethanol verestert zur Bildung von (S)-Norvalinethylester, welchen man bei einem Wasserstoffdruck von 30 bar in Gegenwart von Palladium-auf-Kohlenstoff mit Brenztraubensäure kondensiert, so daß man nach der Kristallisation, dem Abkühlen und der Filtration optisch reines N-[(S)-1-Carbethoxybutyl]-(S)-alanin erhält,
worauf man den in dieser Weise erhaltenen Benzylester der (2S, 3aS, 7aS)-Perhydroindol-2-carbonsäure und das in dieser Weise erhaltene N-[(S)-1-Carbethoxybutyl)-(S)-alanin in alkalischem Medium in Gegenwart von Dicyclohexylcarbodiimid und von 1-Hydroxybenzotriazol kondensiert zur Bildung des entsprechenden Amins, von dem man die Schutzgruppe der Carboxylgruppe des Heterocyclus abspaltet, die Verbindung mit tert.-Butylamin in das Salz überführt und dieses kristallisiert.

3. Verfahren nach Anspruch 1, **dadurch gekennzeichnet, daß** die Konzentration der Verbindung der Formel (I) in Dichlormethan zwischen 100 und 200 g/l liegt.

## Claims

1. Process for the preparation of the β crystalline form of perindopril tert-butylamine salt of formula (I) : 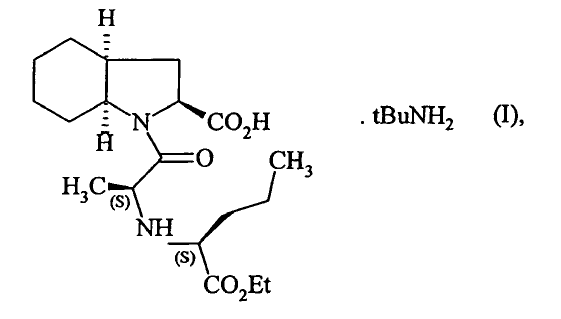 **characterised in that** a solution of perindopril tert-butylamine salt in dichloromethane is heated at reflux, the solution is then cooled to 0°C and the solid obtained is collected by filtration.

2. Process according to claim 1, **characterised in that** the compound of formula (I) that is used is obtained by the following process:
- on the one hand, reduction of the ethyl ester of indole-2-carboxylic acid by the couple tin/hydrochloric acid, followed by a hydrolysis reaction, to yield racemic indoline-2-carboxylic acid, the (S) isomer of which is isolated by addition of the racemate to a solution of (+)-α-methylbenzylamine in ethanol to obtain a precipitate of the salt of (S)-indoline-2-carboxylic acid with α-methylbenzylamine, which, after filtration, is dissolved in water and acidified to yield (S)-indoline-2-carboxylic acid, which, after filtration and washing, is subjected to hydrogenation in the presence of rhodium-on-carbon, under a hydrogen pressure of 30 bars, heating to a temperature of 60°C, to yield, after crystallisation, optically pure (2S, 3aS, 7aS)-perhydroindole-2-carboxylic acid, which is reacted with benzyl alcohol in the presence of para-toluenesulphonic acid to yield the corresponding benzyl ester,
- on the other hand, (S)-L-norvaline is esterified with ethanol to yield ethyl (S)-norvalinate, which is condensed with pyruvic acid under a hydrogen pressure of 30 bars, in the presence of palladium-on-carbon, to yield, after crystallisation, cooling and filtration, optically pure N-[(S)-1-ethoxycarbonylbutyl]-(S)-alanine,
then the benzyl ester of (2S, 3aS, 7aS)-perhydroindole-2-carboxylic acid and N-[(S)-1-ethoxycarbonylbutyl]-(S)-alanine thereby obtained are condensed in an alkaline medium, in the presence of dicyclohexylcarbodiimide and 1-hydroxybenzotriazole, to yield the corresponding amide, which is subjected to deprotection of the carboxylic group of the heterocycle, conversion into a salt using tert-butylamine and crystallisation.

3. Process according to claim 1, **characterised in that** the concentration of the compound of formula (I) in the dichloromethane is from 100 to 200 g/litre.
